# NEUE EUROPÄISCHE PATENTSCHRIFT

(11) **EP 3 630 032 B2**
(45) Veröffentlichungstag und Bekanntmachung des Hinweises auf die Entscheidung über den Einspruch: **10.12.2025**
(45) Hinweis auf die Patenterteilung: 07.07.2021
(21) Anmeldenummer: 18727272.9
(22) Anmeldetag: 24.05.2018
(51) Int. Cl.: A61F 13/08, D02G 3/04, D04B 1/14, D04B 1/26

(54) **KOMPRESSIONSSTRUMPF**
COMPRESSION STOCKING
BAS DE CONTENTION

(30) Priorität: 24.05.2017 DE 102017005187
(43) Veröffentlichungstag der Anmeldung: 08.04.2020
(73) Patentinhaber: Bauerfeind AG, 07937 Zeulenroda-Triebes (DE)
(72) Erfinder: BAUERFEIND, Hans B., 07937 Zeulenroda-Triebes (DE); TUTTE, Andreas, 07955 Auma-Weidatal (DE); THOMÄ, Hans-Jürgen, 07937 Zeulenroda-Triebes (DE)
(74) Vertreter: Wohlfahrt, Jan Günther
(86) Internationale Anmeldenummer: PCT/EP2018/063668
(87) Internationale Veröffentlichungsnummer: WO 2018/215601

(56) Entgegenhaltungen:
- EP-A2- 2 078 518
- EP-A2- 2 078 518
- EP-B1- 2 037 853
- WO-A1-2011/132011
- WO-A1-2011/132011
- WO-A1-2013/138394
- DE-A1- 102006 048 313
- DE-A1- 102006 048 313
- DE-U1- 202015 101 990
- DE-U1- 202015 101 990
- FR-A1- 2 805 459
- FR-A1- 2 805 459
- US-A1- 2011 015 668
- US-A1- 2016 235 354

## Beschreibung

Die vorliegende Erfindung betrifft Kompressionsstrümpfe der Kompressionsklasse I, Verfahren zu deren Herstellung solcher Kompressionsstrümpfe.

Kompressionsstrümpfe, teilweise auch orthopädische Strümpfe oder Stützstrümpfe genannt, sind hinlänglich bekannt, beispielsweise aus der DE 8814047 U1. Sie sind wesentlicher Bestandteil der Kompressionstherapie, beispielsweise bei der Behandlung von Krampfadern, Beinvenenthrombosen, dem postthrombotischen Syndrom, sowie auch von Lymphödemen, "offenen Beinen" und Lipödemen. Kompressionsstrümpfe erzeugen von außen Druck auf das Gewebe des umschlossenen Beins, um dessen geschädigtes Venen- oder Lymphsystem zu entlasten. Ein Kompressionsstrumpf ist so gefertigt, dass der ausgeübte Druck von oben nach unten analog zum Gewebedruck in Richtung der Schwerkraft zunimmt. Dieser Druck ist dem Krankheitsgrad des Patienten angepasst und in die Kompressionsklassen I bis IV eingeteilt. Dabei üben Kompressionsstrümpfe der Kompressionsklasse I eine Kompression von 2,4 bis 2,8 kPa aus, was einem Kompressionsdruck von 18 mmHg bis 21 mmHg entspricht. Kompressionsstrümpfe der Kompressionsklasse II üben eine Kompression von 3,1 bis 4,3 kPa aus, was einen Kompressionsdruck von 23 bis 32 mmHg entspricht. Kompressionsstrümpfe der Kompressionsklasse III üben eine Kompression von 4,5 bis 6,1 kPa aus, was einen Kompressionsdruck von 34 bis 46 mmHg entspricht. Kompressionsstrümpfe der Kompressionsklasse IV üben eine Kompression von mindestens 6,5 kPa aus, was einen Kompressionsdruck von mindestens 49 mmHg entspricht. In schwächerer Ausführung können Kompressionsstrümpfe auch als prophylaktisches Mittel, beispielsweise gegen Reisethrombosen oder als Unterstützung für Angehörige dauernd stehender Berufe Verwendung finden.

Darüber werden Kompressionsstrümpfe in der EP 2 078 518, WO 2011/132011, FR 805459 sowie DE 10 2006 048 313 beschrieben.

Um nahtlos zu sein, wird ein Kompressionsstrumpf meist im Rundstrickverfahren gestrickt. Eine zweite Variante sind flachgestrickte Kompressionsstrümpfe, die nicht im Rundstrickverfahren hergestellt, sondern mit einer Naht wie eine Röhre zusammengenäht werden. Dadurch können die Konturen der Beine wesentlich besser bekleidet werden. Flachgestrickte Kompressionsstrümpfe werden in der Regel bei starken Venenleiden oder Ödemen eingesetzt und haben die Kompressionsklassen II bis IV.

Kompressionsstrümpfe sind in Knie-, Halbschenkel-, Oberschenkel-Länge und ebenso als Strumpfhose ausgeführt. Wahlweise sind sie mit Zeh-Öffnungen oder ohne Zeh-Öffnungen erhältlich.

Bei der peripheren arteriellen Verschlusskrankheit (pAVK), die umgangssprachlich auch Schaufensterkrankheit genannt wird, handelt es sich um eine Störung der arteriellen Durchblutung der Extremitäten. Die Erkrankung gehört zu den chronischen Gefäßkrankheiten der Arterien. Bislang dürfen Kompressionsstrümpfe bei der Behandlung der peripheren arteriellen Verschlusskrankheit nicht verwendet werden, da bei Patienten mit peripherer arterieller Verschlusskrankheit die latente Gefahr besteht, dass man mit von außen aufgebrachter Kompression auf die Blutgefäße deren Erkrankung noch verschlimmert. Es ist daher wünschenswert, Kompressionsstrümpfe bereitzustellen, die auch von pAVK-Patienten getragen werden können.

Kompressionsstrümpfe aus dem Stand der Technik müssen deutlich gedehnt werden, wenn sie über das Bein gezogen werden. Für diese Dehnung benötigt der Benutzer eine entsprechende Kraft oder eine Anziehhilfe.

Das der vorliegenden Erfindung zugrundeliegende technische Problem ist die Bereitstellung eines Kompressionsstrumpfs der Kompressionsklasse I, der beim Anziehen weniger gedehnt werden muss. Bevorzugt soll der erfindungsgemäße Kompressionsstrumpf auch für Menschen, die an der peripheren arteriellen Verschlusskrankheit leiden geeignet sein.

Die Erfindung löst das ihr zugrundeliegende technische Problem durch einen Kompressionsstrumpf nach Anspruch 1.

Die Erfindung betrifft insbesondere einen Kompressionsstrumpf mit einem schlauchförmigen Kompressionsbereich, wobei der Kompressionsbereich einen Kompressionsdruck im Fesselbereich, also Fußfesselbereich, von mindestens 2,4 kPa und höchstens 2,8 kPa ausübt, wobei der Kompressionsbereich aus einem Gestrick mit einem Schussfaden gebildet wird, wobei der Schussfaden einen Kernfaden mit einer Feinheit von mindestens 470 dtex aufweist, und wobei der Kompressionsbereich im Fesselbereich beim Anlegen des Kompressionsstrumpfs um mindestens 5% und höchstens 25% gedehnt ist.

Es zeigte sich überraschenderweise, dass bei der Verwendung eines Schussfadens mit einem Kernfaden mit einer Feinheit von mindestens 470 dtex, also einem für Kompressionsstrümpfe der Kompressionsklasse I, vergleichsweise dicken Schussfaden, ein Kompressionsstrumpf mit einem den Kompressionsbereich bildenden Gestrick erhalten wird, der von seinen Abmessungen den Abmessungen des Beins des Patienten nahezu identisch sein kann, und so der Patient beim Anlegen des Strumpfes bei mehr als 80 % der strumpfbedeckten Beinfläche den Strumpf nicht vordehnen muss, damit dieser über den entsprechenden Beinabschnitt gelegt werden, also angezogen werden kann. Im Vergleich zu den tatsächlich gemessenen Umfangswerten des Patientenbeins können die Umfangswerte vom neuen Maßstrumpf nur sehr gering abweichen. Somit kann der Patient den Kompressionsstrumpf leichter anziehen und es kann eher auf eine Anziehhilfe verzichtet werden.

In vorteilhafter Weise kann bei dem erfindungsgemäßen Kompressionsstrumpf ein herkömmlicher Strickfaden, wie er auch bei Kompressionsstrümpfen aus dem Stand der Technik Anwendung findet, verwendet werden. Auch kann in vorteilhafter Weise die gleiche Stricktechnik, wie bei herkömmlichen Kompressionsstrümpfen verwendet werden. Der Strickfaden kann also ein Gestrick aus dem Stand der Technik bilden, das zusätzlich einen Schussfaden mit einem Kernfaden mit einer Feinheit von mindestens 470 dtex aufweist.

Durch die erfindungsgemäß erhöhte Materialdicke des Kernfadens des Schussfadens beziehungsweise des Schussfadens selbst, ist der Schussfaden unelastischer und benötigt daher weniger Ausdehnung, um die vorgeschriebenen Kompressionswerte zu erreichen. Ein erfindungsgemäß Kompressionsstrumpf mit einem Kompressionsdruck im Fesselbereich von mindestens 2,4 kPa und höchstens 2,8 kPa, also im Bereich der Kompressionsklasse I hat in vorteilhafter Weise bevorzugt eine Kurzzugelastizität, also eine Stiffness, die bei einem vergleichbaren Strumpf der Kompressionsklasse III vorliegt.

Es zeigte sich überraschenderweise, dass sich der erfindungsgemäß Kompressionsstrumpf der Kompressionsklasse I dadurch, dass er in seinem Umfang annähernd dem Umfang des Patientenbeines angepasst werden kann, sich auch für Patienten, die an der peripheren arteriellen Verschlusskrankheit leiden, eignet, und dass darüber hinaus ein erfindungsgemäßer und präzise auf das Patientenbein angepasster Kompressionstrumpf bei solchen Patienten sich sogar auf eine Rückbildung der Symptome des Erkrankungsbildes förderlich auswirkt.

Der erfindungsgemäße Kompressionsstrumpf weist einen schlauchförmigen Kompressionsbereich auf, der wie bei Kompressionsstrümpfen aus dem Stand der Technik im Beinbereich, beispielsweise am Unterschenkel oder am Unter- und Oberschenkel anliegt. Der Kompressionsbereich beginnt unten im Bereich der Fußfessel und zieht sich dann nach oben.

Erfindungsgemäß übt der Kompressionsbereich ein Kompressionsdruck im Fußfesselbereich von mindestens 2,4 kPa und höchstens 2,8 kPa aus, was der Kompressionsklasse I bei Kompressionsstrümpfen entspricht.

Bei der herkömmlichen Messung eines Beins bei der Auswahl geeigneter Kompressionsstrümpfe werden insbesondere der Fesselbereich als Messpunkt B und der Wadenbereich als Messpunkt c herangezogen. Der Messpunkt B gibt den Fesselumfang, also die schlankste Stelle des Unterschenkels und somit der kleinste Beinumfang an. Der Messpunkt C gibt den größten Wadenumfang an. Weitere Messpunkte sind der Messpunkt a als Umfang am Zehenansatz der kleinen Zehe, Messpunkt D, also der Umfang unter der Kniescheibe, insbesondere zwei Finger breit unter der Kniekehle, der Messpunkt E, also der Umfang der Kniescheibe, der Messpunkt F, der in etwa an der Oberschenkelmitte liegt und der Messpunkt G, der in etwa am Ende des Oberschenkels liegt.

Erfindungsgemäß übt der Kompressionsbereich ein Kompressionsdruck im Messpunkt b von mindestens 2,4 kPa und höchstens 2,8 kPa aus.

In einer bevorzugten Ausführungsform ist der Kompressionsstrumpf ein Rundgestrick. In einer bevorzugten Ausführungsform ist der schlauchförmige Kompressionsbereich ein Rundgestrick. In vorteilhafter Weise kann also der erfindungsgemäße Kompressionsstrumpf wie ein herkömmlicher Kompressionsstrumpf als Rundgestrick hergestellt werden. Das Gestrick des Kompressionsbereichs besteht in bevorzugter Ausführungsform aus einem Strickfaden (Grundfaden), der das Gestrick bildet, und dem Schussfaden mit einem Kernfaden mit einer Feinheit, also mit einem Feinheitswert, von mindesten 470 dtex. In vorteilhafter Weise können ein herkömmlicher Strickfaden und eine herkömmliche Stricktechnik verwendet werden.

In einer bevorzugten Ausführungsform weist der Schussfaden einen Kernfaden mit einer Feinheit von mindestens 480 dtex auf. In einer bevorzugten Ausführungsform weist der Schussfaden einen Kernfaden mit einer Feinheit von mindestens 500 dtex auf. In einer bevorzugten Ausführungsform weist der Schussfaden einen Kernfaden mit einer Feinheit von mindestens 550 dtex auf. In einer bevorzugten Ausführungsform weist der Schussfaden einen Kernfaden mit einer Feinheit von mindestens 600 dtex auf. In einer bevorzugten Ausführungsform weist der Schussfaden einen Kernfaden mit einer Feinheit von mehr als 600 dtex auf. In einer bevorzugten Ausführungsform weist der Schussfaden einen Kernfaden mit einer Feinheit von mindestens 605 dtex auf.

In einer bevorzugten Ausführungsform weist der Schussfaden einen Kernfaden mit einer Feinheit, also mit einem Feinheitswert, von höchstens 700 dtex auf. In einer bevorzugten Ausführungsform weist der Schussfaden einen Kernfaden mit einer Feinheit von höchstens 680 dtex auf. In einer bevorzugten Ausführungsform weist der Schussfaden einen Kernfaden mit einer Feinheit von höchstens 650 dtex auf.

In einer bevorzugten Ausführungsform weist der Schussfaden einen Kernfaden mit einer Feinheit von mindestens 605 dtex und höchstens 700 dtex auf. In einer bevorzugten Ausführungsform weist der Schussfaden einen Kernfaden mit einer Feinheit von mindestens 610 dtex und höchstens 650 dtex auf. In einer bevorzugten Ausführungsform weist der Schussfaden einen Kernfaden mit einer Feinheit von mindestens 610 dtex und höchstens 630 dtex auf. In einer bevorzugten Ausführungsform weist der Schussfaden einen Kernfaden mit einer Feinheit von mindestens 615 dtex und höchstens 625 dtex auf.

Im Zusammenhang mit der vorliegenden Erfindung wird unter "eine Feinheit von mindestens x dtex" eine Feinheit verstanden die einem Wert von x dtex oder > x dtex entspricht, also weniger fein ist als x dtex. Im Zusammenhang mit der vorliegenden Erfindung wird unter "eine Feinheit von höchstens x dtex" eine Feinheit verstanden die einem Wert von x dtex oder < x dtex entspricht, also feiner ist als x dtex. Die Angabe der Feinheit bezieht sich also auf den Feinheitswert.

Die erfindungsgemäßen vergleichsweise dicken Schussfäden haben den Vorteil, dass durch den große Feinheitswert des Kernfadens, die zu einem ebenso größeren Feinheitswert des Schussfadens führt, der Schussfaden unelastischer wird und somit weniger Ausdehnung benötigt, um die vorgeschriebenen Kompressionswerte abzubilden.

Die Feinheit des Kernfadens wird in dtex angegeben, also in der Einheit tex gemäß ISO1144 und DIN60905 gemäß dem Gesetz über Einheiten im Messwesen vom 2. Juli 1969. Das tex ist Einheit in Grundgröße des tex-Systems, wobei 1 tex = 1 g Faden pro 1000 m entspricht, also 1 dtex = 1 g / 10.000 m entspricht.

Bevorzugt wird als Prüfverfahren zur Bestimmung der Faserfeinheit ein gravimetrisches Prüfverfahren nach DIN EN ISO 1973 (Wägemethode) verwendet, bei dem Faserbündel, die eine bestimmte Faseranzahl enthalten, auf eine festgelegte Schneidlänge geschnitten und anschließend ausgewogen werden. Alternativ kann als Prüfverfahren das Vibroskopverfahren (Schwingungsverfahren), das ebenfalls in der DIN EN ISO 1973 genormt ist, verwendet werden.

In einer bevorzugten Ausführungsform ist der Kernfaden des Schussfadens elastisch.

Bevorzugt enthält der Kernfaden Elastan oder besteht aus diesem.

In einer bevorzugten Ausführungsform ist der Schussfaden elastisch.

In einer bevorzugten Ausführungsform ist der Kernfaden des Schussfadens umwunden.

Bevorzugt ist der Kernfaden des Schussfadens zweifach umwunden. Bevorzugt enthält der Umwindungsfaden des Schussfadens Polyamid oder besteht aus diesem.

Wenn der Kernfaden nicht umwunden ist, bildet der Kernfaden den Schussfaden.

In einer bevorzugten Ausführungsform weist der Schussfaden einen Feinheitswert von mehr als 605 dtex auf. In einer bevorzugten Ausführungsform weist der Schussfaden einen Feinheitswert von mindestens 650 dtex auf.

In einer bevorzugten Ausführungsform weist der Schussfaden eine Feinheit, also einen Feinheitswert, von mindestens 605 dtex, insbesondere mindestens 700 dtex auf.

Die Angaben der Feinheit des Schussfadens beziehen sich sowohl auf einen umwundenen als auch nicht umwundenen Schussfaden.

Bei einem umwundenen Schussfaden wird die Gesamtdicke des Schussfadens in vorteilhafter Weise nochmals erhöht. Bei einem Schussfaden, bei dem der Kernfaden umwunden ist, wird die Feinheit des Schussfadens, bestehend aus Kernfaden und mindestens einem Umwindungsfaden, bevorzugt nach der französischen Norm bestimmt.

Bevorzugt hat der Schussfaden, insbesondere der umwundene Schussfaden, eine Feinheit von mindestens 750 dtex und höchstens 900 dtex. Bevorzugt hat der Schussfaden, insbesondere umwundene Schussfaden, eine Feinheit von mindestens 750 dtex und höchstens 820 dtex.

Bevorzugt hat der Schussfaden, insbesondere umwundene Schussfaden, eine Feinheit von mindestens 725 dtex.

In einer bevorzugten Ausführungsform weist der Schussfaden ein Kraft-Dehnungs-Verhalten von 0,03 N/cm bis 0,04 N/cm auf, insbesondere im nicht-linearen Bereich.

Durch eine individuelle Herstellung des erfindungsgemäßen Kompressionsstrumpfs kann dieser in vorteilhafter Weise auf die individuellen Beinumfänge des Patienten angepasst werden. Dadurch ist es noch besser möglich, dass der erfindungsgemäße Kompressionsstrumpf auch von pAVK-Patienten getragen werden kann.

Erfindungsgemäß bevorzugt weist der schlauchförmige Kompressionsbereich des Kompressionsstrumpfs in Längsrichtung verschiedene Umfänge auf. Bevorzugt sind die Umfänge dem Beinumfang des Patienten annähernd angepasst. Bevorzugt sind die Umfänge an mindestens fünf Punkten entlang des schlauchförmigen Kompressionsbereichs unterschiedlich, wobei zu diesen fünf Punkten insbesondere der Bereich zählt, der an der Fessel, also Fußfessel anliegt und der Bereich zählt, der an der Wade anliegt, da die Fessel gewöhnlicher Weise den geringsten Umfang des Unterschenkels darstellt und die Wade den größten Umfang des Unterschenkels darstellt.

Erfindungsgemäß ist der Kompressionsbereich im Fesselbereich beim Anlegen des Kompressionsstrumpfs um mindestens 5% und höchstens 25%, bevorzugt höchstens 20 %, gedehnt.

In einer bevorzugten Ausführungsform ist der Kompressionsbereich im Fesselbereich beim Anlegen des Kompressionsstrumpfs um mindestens 15% gedehnt.

In einer bevorzugten Ausführungsform ist der Kompressionsbereich im Wadenbereich, insbesondere beim Messpunkt C beim Anlegen des Kompressionsstrumpfs um höchstens 25 % gedehnt.

In einer bevorzugten Ausführungsform ist der Kompressionsbereich im Wadenbereich beim Anlegen des Kompressionsstrumpfs um mindestens 5% und höchstens 25 % gedehnt.

Neben dem Kompressionsdruck von mindestens 2,4 kPa und höchstens 2,8 kPa, der die Kompressionsklasse I definiert, und der auch als Ruhedruck bezeichnet wird, ist auch der Arbeitsdruck relevant. Er wirkt in erster Linie dynamisch, also wenn sich der Strumpfträger bewegt. Während der Bewegung erzeugen geringfüge anatomische Veränderungen am Bein eine zusätzliche Druckkomponente, eben den Arbeitsdruck. Als technischer Parameter für das Maß des Arbeitsdrucks wird die Stiffness, oder auch Slope-Wert, verwendet. Das europäische Komitee für Normung CEN hat die Stiffness als den Druckanstieg in Millimeter-Quecksilbersäule (mmHg) definiert, der sich bei Überdehnung des Kompressionsstrumpfs um 1 cm ergibt. Da die Stiffness auch vom Umfang des Beines im Messbereich abhängt, wird die Stiffness bevorzugt im Fesselbereich, insbesondere am Messpunkt b, des Beines gemessen. Ein typischer Umfang im Fesselbereich ist etwa 24 cm. Die Stiffness kann aber auch an anderen Messbereichen bestimmt werden, beispielsweise im Wadenbereich. Ein typischer Umfang für die Wade ist etwa 38 cm.

Bei medizinischen Kompressionsstrümpfen ist der Arbeitsdruck ein nicht zu vernachlässigender Faktor. Kompressionsstrümpfe mit einer hohen Stiffness benötigen eine hohe Anforderung an die Passgenauigkeit, da das Kurzzugmaterial, aus dem solche Kompressionsstrümpfe bestehen, nur sehr geringe Mess-Toleranzen erlaubt.

Es zeigte sich nun überraschender Weise, dass ein erfindungsgemäßer Kompressionsstrumpf in vorteilhafter Weise eine erhöhte Stiffness aufweisen kann und dabei passgenau gefertigt sein kann. Die erhöhte Stiffness führt in vorteilhafter Weise zu einem höheren Arbeitsdruck bei gleichzeitig geringem Ruhedruck, beispielsweise einem Kompressionsdruck von mindestens 2,4 kPa und höchstens 2,8 kPa, der die Kompressionsklasse I definiert. Ein höher Arbeitsruck bei niedrigem Ruhedruck erlaubt in überraschender und vorteilhafter Weise die Verwendung des erfindungsgemäßen Kompressionsstrumpfs bei pAVK-Patienten, da der Kompressionsstrumpf im Ruheszustand nahezu keinen Druck aufbaut, während er bei Dehnung durch die Bewegung des Beins den erforderlichen Druck bereitstellt.

In einer bevorzugten Ausführungsform weist der schlauchförmige Kompressionsbereich bei einem Umfang von 24 cm eine Stiffness mindestens 5 mmHg/cm und höchstens 12 mmHg/cm auf.

In einer bevorzugten Ausführungsform weist der schlauchförmige Kompressionsbereich bei einem Umfang von 24 cm eine Stiffness mindestens 6 mmHg/cm und höchstens 12 mmHg/cm auf. In einer bevorzugten Ausführungsform weist der schlauchförmige Kompressionsbereich bei einem Umfang von 24 cm eine Stiffness von mindestens 6 mmHg/cm, bevorzugt von mindestens 8 mmHg/cm auf.

Besonders bevorzugt weist der schlauchförmige Kompressionsbereich bei einem Umfang von 24 cm eine Stiffness von mindestens 8 mmHg/cm und höchstens 10 mmHg/cm auf.

Besonders bevorzugt weist der schlauchförmige Kompressionsbereich bei einem Umfang von 24 cm eine Stiffness von mindestens 6 mmHg/cm und höchstens 11 mmHg/cm auf.

In einer bevorzugten Ausführungsform weist der schlauchförmige Kompressionsbereich im Fesselbereich, insbesondere am Messpunkt B, eine Stiffness von mindestens 6 mmHg/cm und höchstens 12 mmHg/cm auf. In einer bevorzugten Ausführungsform weist der schlauchförmige Kompressionsbereich im Fesselbereich, insbesondere am Messpunkt B, eine Stiffness von mindestens 6 mmHg/cm auf.

In einer bevorzugten Ausführungsform weist der schlauchförmige Kompressionsbereich bei einem Umfang von 38 cm eine Stiffness von mindestens 0,5 mmHg auf. In einer bevorzugten Ausführungsform weist der schlauchförmige Kompressionsbereich bei einem Umfang von 38 cm eine Stiffness von mindestens 0,5 mmHg und höchstens 6 mmHg auf.

In einer bevorzugten Ausführungsform weist der schlauchförmige Kompressionsbereich bei einem Umfang von 38 cm eine Stiffness von mindestens 0,5 mmHg und höchstens 5 mmHg auf.

In einer bevorzugten Ausführungsform weist der schlauchförmige Kompressionsbereich im Wadenbereich, insbesondere am Messpunkt C, eine Stiffness von mindestens 0,5 mmHg/cm und höchstens 5 mmHg/cm auf. In einer bevorzugten Ausführungsform weist der schlauchförmige Kompressionsbereich im Wadenbereich, insbesondere am Messpunkt C, eine Stiffness von mindestens 0,5 mmHg/cm auf.

In einer bevorzugten Ausführungsform hat der Kompressionsstrumpf eine kompressionsreduzierte Ferse, insbesondere in Form eines Fersenbeutels. Diese ist bevorzugt ohne Kompression gestrickt und vergleichsweise dehnungsfähig. Dabei sind bevorzugt die fersenbildenden Maschen im Vergleich zu den Maschen des Kompressionsbereichs bevorzugt im Verhältnis von etwa 60 zu 40 gestrickt, der Fersenbereich enthält also pro Abschnitt mehr Maschen als der Kompressionsbereich. Dadurch wird der "Fersenbeutel" etwas größer und der Einstieg in den Strumpf komfortabler. Dies führt aber auch dazu, dass man in der ersten Maschenreihe nach dem Fersenbeutel im Bereich des Spanns einen harten Übergang zum zirkulären Kompressionsbereich erzeugt. Druck an dieser Stelle kann als unangenehm empfunden werden. Daher hat der Kompressionsstrumpf bevorzugt einen Übergangsbereich eingestrickt, bevorzugt in einer Breite von etwa 2 cm, der zu beiden Nachbarbereichen in vorteilhafter Weise einen vermittelnden Kompressionswert ausbildet.

In einer bevorzugten Ausführungsform ist der Kompressionsstrumpf individuell hergestellt. Dadurch kann in vorteilhafter Weise der Umfang des Kompressionsbereichs an die verschiedenen Messpunkte am Bein individuell angepasst werden, so dass eine bestmögliche Passform erreicht wird und der Kompressionsstrumpf sich besonders gut auch für pAVK-Patienten eignet.

Die Erfindung betrifft auch ein Verfahren zum Herstellen eines erfindungsgemäßen Kompressionsstrumpfes, aufweisend die Schritte:
a) Messen von mindestens 5 Umfangsmaßen eines Unterschenkels oder eines Beins,
b) Stricken des erfindungsgemäßen aus einem Strickfaden und dem Schussfaden, wobei der schlauchförmige Kompressionsbereich des Kompressionsstrumpf unterschiedliche Umfangsmaße aufweist, die auf den gemessenen mindestens 5 Umfangsmaßen aus Schritt a) basieren.

In einer bevorzugten Ausführungsform weist das erfindungsgemäße Verfahren die Schritte auf:
a) Messen von mindestens 5 Umfangsmaßen eines Unterschenkels oder eines Beins,
b) Interpolieren der mindestens 5 Umfangsmaße;
c) Stricken des erfindungsgemäßen Kompressionsstrumpfes aus einem Strickfaden und dem Schussfaden, wobei der Kompressionsstrumpf unterschiedliche Umfangsmaße aufweist, die auf den interpolierten Umfangmaßen aus Schritt b) basieren.

Zwischen den einzelnen Messpunkten werden also bevorzugt interpolierte Werte zum Stricken des Kompressionsstrumpfes herangezogen.

Bevorzugte ausführungsformen des Verfahrens ergeben sich auch aus den bevorzugten Ausführungsformen des Kompressionsstrumpfes.

Erfindungsgemäß bevorzugt werden mindestens 5 Umfangsmaße des Unterschenkels oder mindestens 10 Umfangsmaße des Beins gemessen, wobei bevorzugt mehr als 5 Umfangsmaße beziehungsweise 10 Umfangsmaße gemessen werden. Beispielsweise können mit dem Messsystem Bauerfeind Bodytronic^{®} 600 20 Messwerte und mehr abgenommen werden, was in vorteilhafter Weise zu einem noch passgenaueren Kompressionsstrumpf führt. In einer besonderen Ausführungsform des erfindungsgemäßen Verfahrens werden mindestens 20 Umfangsmaße des Unterschenkels oder des Beins gemessen.

Die Erfindung betrifft auch einen Kompressionsstrumpf der Kompressionsklasse I, hergestellt und/oder herstellbar durch das erfindungsgemäße Verfahren.

Die Erfindung betrifft auch die Verwendung eines erfindungsgemäßen Kompressionsstrumpf der Kompressionsklasse I, zur Behandlung einen Beinleidens oder zur Vorbeugung vor einem Beinleiden, wobei das Beinleiden insbesondere eine periphere arterielle Verschlusskrankheit sein kann.

Bevorzugte Ausführungsformen ergeben sich auch aus den Unteransprüchen.

Die Erfindung wird anhand des folgenden Beispiels und der Figuren näher erläutert, ohne dass diese einschränkend zu verstehen wären.
- Figur 1: zeigt eine bevorzugte Ausführungsform eines erfindungsgemäßen Kompressionsstrumpfs;
- Figur 2: zeigt einen Ausschnitt des Kompressionsbereichs des Kompressionstrumpfs aus Figur 1.

### BEISPIEL

Der Umfang eines Beins eines Probanden wurde an fünf Messpunkten gemessen, unter anderem an der Fessel (Messpunkt B, Höhe, gemessen jeweils von der Ferse, 16,7 cm) und an der Wade (Messpunkt C, Höhe 36,5 cm). Der Umfang am Messpunkt B betrug 24,2 cm, der Umfang am Messpunkt C 36,5 cm. Weiterhin betrug der Umfang am Messpunkt B1 (Höhe 26,3 cm) 29,8 cm, am Messpunkt B2 (Höhe 31,2 cm) 34,4 cm und am Messpunkt D (Höhe 41,7 cm) 34,9 cm.

Ein Kompressionsstrumpf der Kompressionsklasse I in Halbschenkel-Länge wurde auf einer herkömmlichen Rundstrickmaschine (Einzylinder Rundstrickmaschine Durchmesser 3,5" bis 6") aus einem Strickfaden und einem Schussfaden gestrickt. Der Strickfaden bildete dabei in herkömmlicher Weise das den schlauchförmigen Kompressionsbereich bildenden Rundgestrick. Bei dem Strickfaden handelte es sich um einen Strickfaden, wie er auch bei herkömmlichen Kompressionsstrümpfen Verwendung findet. In das Gestrick, das den schlauchförmigen Kompressionsbereich bildet wurde ein Schussfaden eingelegt. Der Schussfaden war ein doppelumwundener Schussfaden, dessen Kernfaden aus Elastan bestand und eine Feinheit von 620 dtex aufwies (Lycra^{®} 620 dtex Typ 136c). Die Umwindungsfäden bestanden aus Polyamid (44f13x2 PA 6.6 ME). Der Schussfaden hatte eine Feinheit, gemessen nach französischer Norm, von 790 dtex. Der Kernfaden hatte im Kraft-Dehnungsverhalten einen Anstieg von 0,03133 N/cm. Der umwundene Schussfaden hatte im Kraft-Dehnungsverhalten einen Anstieg von 0,03890 N/cm.

Der so gestrickte Kompressionsstrumpf wies im Kompressionsbereich folgende Parameter auf:
a) Ruhedruck (RAL GZ-387):
   Messpunkt B: 2,57 kPa
   Messpunkt B1: 2,06 kPa
   Messpunkt B2: 1,99 kPa
   Messpunkt C: 1,55 kPa
   Messpunkt D: 1,85 kPa.
b) Dehnung beim angezogenen Strumpf:
   Messpunkt B: 5% bis 20%
   Messpunkt C: 5% bis 25%
c) Arbeitsdruck:
   Messpunkt B: Slope-Wert: ca. 9,0 mmHg/cm
   Messpunkt C: Slope-Wert: ca. 2,0 mmHg/cm

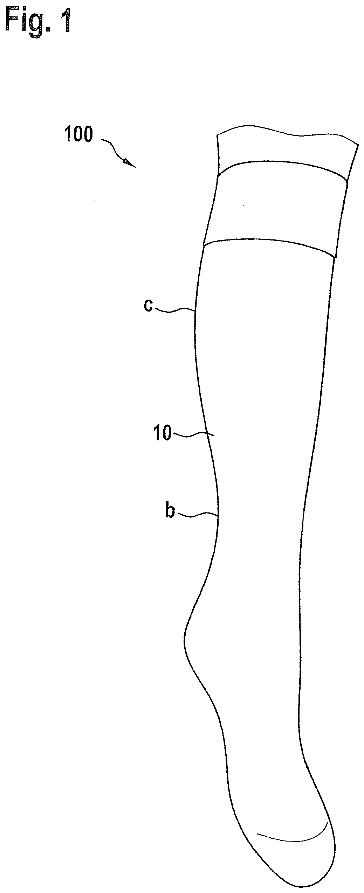

Figur 1 zeigt den erfindungsgemäßen Kompressionsstrumpf (100) aus dem Beispiel im angezogenen Zustand. Der Kompressionsstrumpf (100) weist einen schlauchförmigen Kompressionsbereich (10) mit den Messpunkten B und C auf.

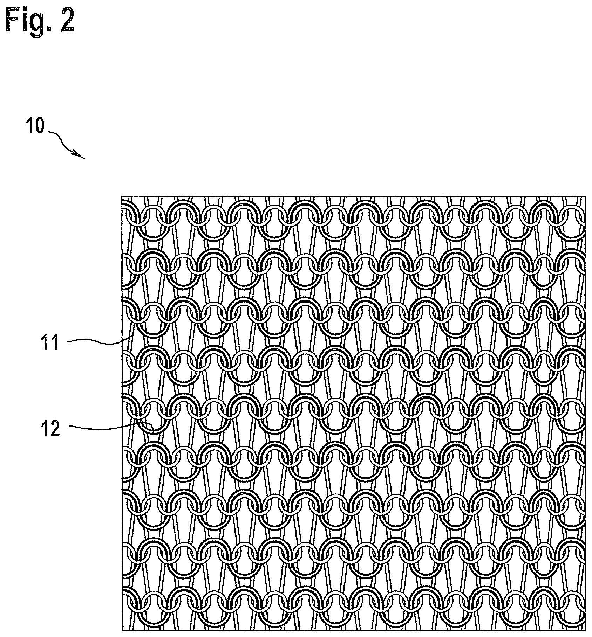

Figur 2 zeigt einen Ausschnitt aus dem Kompressionsbereich (10) des Kompressionsstrumpfs aus Figur 1 in gedehntem Zustand. Der Kompressionsbereich (10) wird aus dem aus einem Strickfaden (11) gebildeten Gestrick gebildet, in das der vergleichsweise dicke Schussfaden (12) eingelegt ist. gehenden Ansprüche, wobei der Kompressionsbereich im Fesselbereich beim Anlegen des Kompressionsstrumpfs um mindestens 5% und höchstens 25% gedehnt ist.

## Patentansprüche

1. Kompressionsstrumpf (100) mit einem schlauchförmigen Kompressionsbereich (10), wobei der Kompressionsbereich (10) aus einem Gestrick (11) mit einem Schussfaden (12) gebildet wird, **dadurch gekennzeichnet, dass** der Schussfaden einen Kernfaden mit einer Feinheit von mindestens 470 dtex aufweist, wobei der Kompressionsbereich (10) einen Kompressionsdruck im Fesselbereich von mindestens 2,4 kPa und höchstens 2,8 kPa ausübt, wobei der Kompressionsbereich im Fesselbereich beim Anlegen des Kompressionsstrumpfs um mindestens 5% und höchstens 25% gedehnt ist.

2. Kompressionsstrumpf (100) nach einem der vorhergehenden Ansprüche, wobei der Schussfaden einen Kernfaden mit einer Feinheit von mindestens 605 dtex und höchstens 700 dtex aufweist.

3. Kompressionsstrumpf (100) nach einem der vorhergehenden Ansprüche, wobei der Kernfaden des Schussfadens Elastan enthält oder aus diesem besteht.

4. Kompressionsstrumpf (100) nach einem der vorhergehenden Ansprüche, wobei der Kernfaden des Schussfadens umwunden ist.

5. Kompressionsstrumpf (100) nach einem der vorhergehenden Ansprüche, wobei der Schussfaden eine Feinheit von mindestens 605 dtex, insbesondere mindestens 700 dtex aufweist.

6. Kompressionsstrumpf (100) nach einem der vorhergehenden Ansprüche, wobei der Schussfaden ein Kraft-Dehnungs-Verhalten von 0,03 N/cm bis 0,04 N/cm aufweist.

7. Kompressionsstrumpf (100) nach einem der vorhergehenden Ansprüche, wobei der schlauchförmige Kompressionsbereich bei einem Umfang von 24 cm eine Stiffness mindestens 6 mmHg/cm und höchstens 12 mmHg/cm aufweist.

8. Kompressionsstrumpf (100) nach einem der vorhergehenden Ansprüche, wobei der Kompressionsstrumpf individuell hergestellt ist.

9. Verfahren zum Herstellen eines Kompressionsstrumpfes nach einem der Ansprüche 1 bis 8, aufweisend die Schritte:
a) Messen von mindestens 5 Umfangsmaßen eines Unterschenkels oder eines Beins,
b) Stricken des Kompressionsstrumpfes nach einem der Ansprüche 1 bis 8 aus einem Strickfaden und dem Schussfaden, wobei der schlauchförmige Kompressionsbereich des Kompressionsstrumpfs unterschiedliche Umfangsmaße aufweist, die auf den gemessenen mindestens 5 Umfangsmaßen aus Schritt a) basieren.

10. Verfahren nach Anspruch 9, aufweisend die Schritte:
a) Messen von mindestens 5 Umfangsmaßen eines Unterschenkels oder mindestens 10 Umfangsmaßen eines Beins,
b) Interpolieren der mindestens 5 Umfangsmaße oder der mindestens 10 Umfangsmaße;
c) Stricken des Kompressionsstrumpfes nach einem der Ansprüche 1 bis 8 aus einem Strickfaden und dem Schussfaden, wobei der Kompressionsstrumpf unterschiedliche Umfangsmaße aufweist, die auf den interpolierten Umfangmaßen aus Schritt b) basieren.

## Claims

1. A compression stocking (100) having a tubular compression region (10), wherein the compression region (10) is formed from a knitted fabric (11) having a weft thread (12), **characterized in that** the weft thread has a core thread having a fineness of at least 470 dtex, wherein the compression region (10) in the ankle region exerts a compression pressure of at least 2.4 kPa and at most 2.8 kPa, wherein the compression region in the ankle region when putting on the compression stocking is elongated by at least 5% and at most 25%.

2. The compression stocking (100) as claimed in claim 1, wherein the weft thread has a core thread having a fineness of at least 605 dtex and at most 700 dtex.

3. The compression stocking (100) as claimed in one of the preceding claims, wherein the core thread of the weft thread comprises elastane or is composed thereof.

4. The compression stocking (100) as claimed in one of the preceding claims, wherein the core thread of the weft thread is wrapped.

5. The compression stocking (100) as claimed in one of the preceding claims, wherein the weft thread has a fineness of at least 605 dtex, in particular 700 dtex.

6. The compression stocking (100) as claimed in one of the preceding claims, wherein the weft thread has a force/elongation behavior of 0.03 N/cm to 0.04 N/cm.

7. The compression stocking (100) as claimed in one of the preceding claims, wherein the tubular compression region at a circumference of 24 cm has a stiffness of at least 6 mmHg/cm and at most 12 mmHg/cm.

8. The compression stocking (100) as claimed in one of the preceding claims, wherein the compression stocking is individually produced.

9. A method for producing a compression stocking as claimed in one of claims 1 to 8, comprising the following steps:
a) measuring at least 5 circumferential measurements of a lower leg or of a leg;
b) knitting the compression stocking as claimed in one of claims 1 to 8 from a knitting thread and the weft thread, wherein the tubular compression region of the compression stocking has dissimilar circumferential measurements which are based on the at least 5 measured circumferential measurements from step a).

10. The method as claimed in claim 9, comprising the following steps:
a) measuring at least 5 circumferential measurements of a lower leg, or at least 10 circumferential measurements of a leg;
b) interpolating the at least 5 circumferential measurements or the at least 10 circumferential measurements;
c) knitting the compression stocking as claimed in one of claims 1 to 10 from a knitting thread and the weft thread, wherein the compression stocking has dissimilar circumferential measurements which are based on the interpolated circumferential measurements from step b).

## Revendications

1. Bas de compression (100) avec une région de compression tubulaire (10), dans lequel la région de compression (10) est formée d'un tricotage (11) avec un fil de trame (12), **caractérisé en ce que** le fil de trame présente un fil d'âme avec une finesse d'au moins 470 dtex, dans lequel la région de compression (10) exerce une pression de compression dans la région de la cheville d'au moins 2,4 kPa et d'au plus 2,8 kPa, dans lequel la région de compression dans la région de la cheville est allongée d'au moins 5 % et d'au plus 25 % lors de l'application du bas de compression.

2. Bas de compression (100) selon une des revendications précédentes, dans lequel le fil de trame présente un fil d'âme avec une finesse d'au moins 605 dtex et d'au plus 700 dtex.

3. Bas de compression (100) selon une des revendications précédentes, dans lequel le fil d'âme du fil de trame contient de l'élasthanne ou se compose de celle-ci.

4. Bas de compression (100) selon une des revendications précédentes, dans lequel le fil d'âme du fil de trame est enlacée.

5. Bas de compression (100) selon une des revendications précédentes, dans lequel le fil de trame présente une finesse d'au moins 605 dtex, notamment d'au moins 700 dtex.

6. Bas de compression (100) selon une des revendications précédentes, dans lequel le fil de trame présente un comportement de contrainte-allongement de 0,03 N/cm à 0,04 N/cm.

7. Bas de compression (100) selon une des revendications précédentes, dans lequel la région de compression tubulaire présente à une circonférence de 24 cm une rigidité d'au moins 6 mmHg/cm et d'au plus 12 mmHg/cm.

8. Bas de compression (100) selon une des revendications précédentes, dans lequel le bas de compression est fabriqué individuellement.

9. Procédé de fabrication d'un bas de compression selon une des revendications 1 à 8, présentant les étapes de :
a) mesure d'au moins 5 dimensions circonférentielles du bas d'une jambe ou d'une jambe,
b) tricotage du bas de compression selon une des revendications 1 à 8 à partir d'un fil à tricoter et du fil de trame, dans lequel la région de compression tubulaire du bas de compression présente différentes dimensions circonférentielles qui se basent sur les au moins 5 dimensions circonférentielles mesurées de l'étape a).

10. Procédé selon la revendication 9, présentant les étapes de :
a) mesure d'au moins 5 dimensions circonférentielles du bas d'une jambe ou d'au moins 10 dimensions circonférentielles d'une jambe,
b) interpolation des au moins 5 dimensions circonférentielles ou des au moins 10 dimensions circonférentielles ;
c) tricotage du bas de compression selon une des revendications 1 à 10 à partir d'un fil à tricoter et du fil de trame, dans lequel la région de compression présente différentes dimensions circonférentielles qui se basent sur les dimensions circonférentielles interpolées de l'étape b).
